# EUROPEAN PATENT SPECIFICATION

(11) **EP 1 909 742 B1**
(45) Date of publication and mention of the grant of the patent: **14.07.2010**
(21) Application number: 06776521.4
(22) Date of filing: 31.07.2006
(51) Int. Cl.: A61K 6/027, A61K 6/083, C03C 17/32

(54) **DENTAL COMPOSITIONS CONTAINING A SURFACE-MODIFIED FILLER**
DENTALZUSAMMENSETZUNGEN MIT EINEM OBERFLÄCHENMODIFIZIERTEN FÜLLSTOFF
COMPOSITIONS DENTAIRES CONTENANT UNE CHARGE A SURFACE MODIFIEE

(30) Priority: 05.08.2005 US 706180 P
(43) Date of publication of application: 16.04.2008
(73) Proprietor: 3M ESPE AG, 82229 Seefeld (DE)
(72) Inventor: ECKHARDT, Gunther, 06231 Bad Dürrenberg (DE); MIKULLA, Markus, 82346 Andechs-Frieding (DE); PEEZ, Robert, 86899 Landsberg (DE); BISSINGER, Peter, 86911 Diessen (DE); RACKELMANN, Gabriele, 84561 Mehring (DE)
(74) Representative: Brem, Roland
(86) International application number: PCT/EP2006/007557
(87) International publication number: WO 2007/017152

(56) References cited:
- EP-A- 1 600 142
- EP-A1- 0 694 298
- WO-A-98/38967

## Description

### Field of the Invention

The present invention relates to hardenable dental compositions. More specifically, the invention relates to ionomer and resin-modified ionomer compositions containing at least one filler that has been surface-modified with a polyacid. The compositions can be used in a variety of applications, for example, as adhesives, cements, restoratives, coatings, and sealants.

### Background

The restoration of decayed dental structures including caries, decayed dentin or decayed enamel, is often accomplished by the sequential application of a dental adhesive and then a dental material (e.g., a restorative material) to the relevant dental structures. Similar compositions are used in the bonding of orthodontic appliances (generally utilizing an orthodontic adhesive) to a dental structure. Often various pretreatment processes are used to promote the bonding of adhesives to dentin or enamel. Typically, such pretreatment steps include etching with, for example, inorganic or organic acids, followed by priming to improve the bonding between the tooth structure and the overlying adhesive.

A variety of dental and orthodontic adhesives, cements, and restoratives are currently available. Compositions including fluoroaluminosilicate glass fillers (also known as glass ionomer or "GI" compositions) are among the most widely used types of dental materials. These compositions have a broad range of applications such as filling and restoration of carious lesions; cementing of, for example, a crown, an inlay, a bridge, or an orthodontic band; lining of a cavity; core construction; and pit and fissure sealing.

There are currently two major classes of glass ionomers. The first class, known as conventional glass ionomers, generally contains as main ingredients a homopolymer or copolymer of an α,β-unsaturated carboxylic acid, a fluoroaluminosilicate ("FAS") glass, water, and optionally a chelating agent such as tartaric acid. These conventional glass ionomers typically are supplied in powder/liquid formulations that are mixed just before use. The mixture undergoes self-hardening in the dark due to an ionic acid-base reaction between the acidic repeating units of the polycarboxylic acid and cations leached from the basic glass.

The second major class of glass ionomers is known as hybrid glass ionomers or resin-modified glass ionomers ("RMGI"). Like a conventional glass ionomer, an RMGI employs an FAS glass An RMGI also contains a homopolymer or copolymer of an α,β-unsaturated carboxylic acid, an FAS glass, and water; however, the organic portion of an RMGI is different. In one type of RMGI, the polyacid is modified to replace or end-cap some of the acidic repeating units with pendent curable groups and a photoinitiator is added to provide a second cure mechanism. Acrylate or methacrylate groups are typically employed as the pendant curable group. In another type of RMGI, the composition includes a polycarboxylic acid, an acrylate or methacrylate-functional monomer or polymer, and a photoinitiator. The polyacid may optionally be modified to replace or end-cap some of the acidic repeating units with pendent curable groups. A redox or other chemical cure system may be used instead of or in addition to a photoinitiator system. RMGI compositions are usually formulated as powder/liquid or paste/paste systems, and contain water as mixed and applied. They may partially or fully harden in the dark due to the ionic reaction between the acidic repeating units of the polycarboxylic acid and cations leached from the glass, and commercial RMGI products typically also cure on exposure of the cement to light from a dental curing lamp.

There are many important benefits provided by glass ionomer compositions. For example, fluoride release from glass ionomers tends to be higher than from other classes of dental compositions such as metal oxide cements, compomer cements, or fluoridated composites, and thus glass ionomers are believed to provide enhanced cariostatic protection. Another advantage of glass ionomer materials is the very good clinical adhesion of such cements to tooth structure, thus providing highly retentive restorations. Since conventional glass ionomers do not need an external curing initiation mode, they can generally be placed in bulk as a filling material in deep restorations, without requiring layering.

One of the drawbacks of conventional glass ionomers is that these compositions are somewhat technique sensitive when mixed by hand. They are typically prepared from a powder component and a liquid component, thus requiring weighing and mixing operations prior to application. The accuracy of such operations depends in part on operator skill and competency. When mixed by hand, the powder component and the liquid component are usually mixed on paper with a spatula. The mixing operation must be carried out within a short period of time, and a skilled technique is needed in order for the material to fully exhibit the desired characteristics (i.e., the performance of the cement can depend on the mixture ratio and the manner and thoroughness of mixing). Alternatively, some of these inconveniences and technique sensitivities have been improved by utilization of powder liquid capsule dispensing systems that contain the proper proportion of the powder and liquid components. While capsules provide proper proportions of the powder and liquid components, they still require a capsule activation step to combine the two components followed by mechanical mixing in a dental triturator.

Conventional glass ionomers may also be quite brittle as evidenced by their relatively low flexural strength. Thus, restorations made from conventional glass ionomers tend to be more prone to fracture in load bearing indications.. In addition, glass ionomers are often characterized by high visual opacity (i.e., cloudiness), especially when they come into contact with water at the initial stage of hardening, resulting in relatively poor aesthetics.

Cured RMGIs typically have increased strength properties (e.g., flexural strength), are less prone to mechanical fracture than conventional glass ionomers, and typically require a primer or conditioner for adequate tooth adhesion. In addition, RMGIs typically include 2-hydroxyethyl methacrylate ("HEMA") as a reactive diluent, which acts as a compatibilizer. HEMA makes it possible to mix the water with the reactive (meth)acrylates, such as GDMA (Glycerol Dimethacrylate), UDMA (Diurethane Dimethacrylate), etc., that are present in the RMGI composition. Thus it is possible to obtain a one-phase system that hardens after mixing without separation of the GI salt matrix and the resin matrix. However, the use of HEMA leads to some noticeable disadvantages of RMGIs. For example, it is well known that the HEMA can lead to discoloration of the material, thereby reducing its aesthetic properties.

EP 1 600 142 A1 features a dental cement composition comprising (i) a particulate reactive inorganic filler capable of leaching metal ions in the presence of an acid and (ii) composite particles with certain grafted polyacidic polymer chains. Further polymerizable monomers may be incorporated into the dental cement composition in an amount of up to 20 percent by weight.

EP 0 694 298 A1 describes a fluoride ion sustained release pre-formed glass ionomer filler comprised of a powder-form reaction product of polyalkenoic acid and a fluorine-containing glass.

WO 98/38967 A1 relates to a hydrogel material, which comprises a continuous matrix phasee of fluoride-containing gel and a dispersed phase of non-fluoride silica gel, which is the product of reacting to completion an acid-degradable glass with an acid polymer by an acid-base cement-forming reaction.

### Summary

The present invention as defined in claim 1 provides hardenable dental compositions, including stable GI and RMGI compositions, containing a filler that has been surface-modified to include a polyacid. This polyacid is attached to the filler and acts a compatibilizer, thus enabling the formulation of, for example, RMGI compositions that do not require the presence of HEMA. In one embodiment, the present invention features a hardenable dental composition comprising a polymerizable component and a filler that has been surface-modified with a first polyacid. The polymerizable component is an ethylenically unsaturated compound, optionally with acid functionality. In addition, the composition typically includes a second polyacid, an acid-reactive filler, and water.

The composition of the invention includes at least one filler that has been surface-modified with a first polyacid. Preferably, the first polyacid is bonded to the filler surface by covalent bonds, but may be attached to the filler in other ways, e.g. via an ionic bond. The polyacid may be attached directly to the surface of the filler, or may be attached to a linking group that is directly attached to the surface of the filler. Suitable linking groups include aminoalkyltrialkoxysilanes in which the silane atom is attached directly (e.g., through covalent bonding) to the filler surface and in which the amino moiety is attached directly (e.g., through an amide linkage) to the polyacid.

Suitable polyacids that can be attached to a filler include homopolymers or copolymers of acrylic acid, maleic acid, itaconic acid, methacrylic acid, e.g., a copolymer of acrylic acid and another α-β unsaturated carboxylic acid, e.g. maleic or itaconic acid. Other especially suitable polyacids include, e.g., methacrylic acid, etc., or polyacids substantially free of polymerizable groups, or alternatively comprising a plurality of polymerizable groups (e.g., VITREBOND copolymer).

The second polyacid component of the composition typically comprises a polymer having a plurality of acidic repeating groups. The acidic repeating groups can be acids of carbon (e.g., carboxylic acids), sulfur (e.g., sulfuric and sulfonic acids), phosphorus (e.g., phosphoric and phosphonic acids), or combinations thereof. In certain embodiments, the acidic repeating groups are carboxylic acids. Typical embodiments include polymers and copolymers of acrylic acid, maleic acid, itaconic acid and methacrylic acid. The polymer may be substantially free of polymerizable groups, or alternatively it may comprise a plurality of polymerizable groups. The second polyacid and the ethylenically unsaturated compound can be the same.

The first polyacid and the second polyacid can be the same. The second polyacid and the ethylenically unsaturated compound can be the same. The first polyacid, the second polyacid, and the ethylenically unsaturated compound can all be the same.

The acid-reactive filler component of the composition is generally selected from metal oxides, glasses, metal salts, and combinations thereof. Typically, the acid-reactive filler comprises an FAS glass. One of the advantages of certain embodiments of the present invention is that a hardenable composition may be prepared with less acid-reactive filler than previous GI and RMGI compositions. Accordingly, in one embodiment, the composition of the invention comprises less than 50 percent by weight acid-reactive filler, typically an FAS glass.

In another embodiment of the invention, the acid-reactive filler comprises an oxyfluoride material, which is typically nanostructured, e.g., provided in the form of nanoparticles. Generally, the acid-reactive oxyfluoride material is non-fused and includes at least one trivalent metal (e.g., aluminum, lanthanum, etc.), oxygen, fluorine, and at least one alkaline earth metal (e.g. strontium, calcium, barium, etc.). The oxyfluoride material may be in the form of a coating on particles or nanoparticles, such as metal oxide particles (e.g., silica).

The fillers used in the present invention may be, for example, nanofillers which may be either acid reactive or non-acid reactive. Typically, the nanofiller comprises nanoparticles selected from silica; zirconia; oxides of titanium, aluminum, cerium, tin, yttrium, strontium, barium, lanthanum, zinc, ytterbium, bismuth, iron, and antimony; and combinations thereof. Often a portion of the surface of the nanofiller is silane treated or otherwise chemically treated to provide one or more desired physical properties.

The compositions of the invention may also include one or more optional additives, such as, for example, other fillers, pyrogenic fillers, fluoride sources, whitening agents, anticaries agents (e.g., xylitol), remineralizing agents (e.g., calcium phosphate compounds), enzymes, breath fresheners, anesthetics, clotting agents, acid neutralizers, chemotherapeutic agents, immune response modifiers, medicaments, indicators, dyes, pigments, wetting agents, tartaric acid, chelating agents, surfactants, buffering agents, viscosity modifiers, thixotropes, polyols, antimicrobial agents, anti-inflammatory agents, antifungal agents, stabilizers, agents for treating xerostomia, desensitizers, and combinations thereof.

The compositions of the invention may further include a photoinitiator system and/or a redox cure system.

Additionally, the compositions may be provided in the form of a multi-part system in which the various components are divided into two or more separate parts. Typically, the composition is a two-part system, such as a paste-paste composition, a paste-liquid composition, a paste-powder composition, or a powder-liquid composition.

As discussed above, one of the features of certain embodiments of the present invention is providing hardenable ionomer compositions while using less acid-reactive filler than conventional glass ionomers. This facilitates the preparation of a two-part, paste-paste composition, which is generally desirable because of the ease of mixing and dispensing of such a system compared to, for example, a powder-liquid system.

Compositions according to the invention are useful in a variety of dental and orthodontic applications, including dental restoratives, dental adhesives, dental cements, cavity liners, orthodontic adhesives, dental sealants, and dental coatings. The compositions may be used to prepare a dental article by hardening to form, for example, dental mill blanks, dental crowns, dental fillings, dental prostheses, and orthodontic devices.

The ionomer compositions of the invention generally exhibit good aesthetics, low visual opacity, good wear properties, good physical properties including mechanical strengths, e.g., flexural, diametral tensile and compressive strengths, and good adhesive strength to tooth structures. In addition, the invention provides for easy mixing and convenient dispensing options made possible by formulation of a paste-paste composition.

Other features and advantages of the present invention will be apparent from the following detailed description thereof, and from the claims.

### Definitions

By "hardenable" is meant that the composition can be cured or solidified, e.g. by heating, chemical cross-linking, radiation-induced polymerization or crosslinking, or the like.

By "filler" is meant a particulate material suitable for use in the oral environment. Dental fillers generally have an average particle size of at most 100 micrometers.

By "nanofiller" is meant a filler having an average primary particle size of at most 200 nanometers. The nanofiller component may be a single nanofiller or a combination of nanofillers. Typically the nanofiller comprises non-pyrogenic nanoparticles or nanoclusters.

By "paste" is meant a soft, viscous mass of solids dispersed in a liquid.

By "acid-reactive filler" is meant a filler that chemically reacts in the presence of an acidic component.

By "oxyfluoride" is meant a material in which atoms of oxygen and fluorine are bonded to the same atom (e.g., aluminum in an aluminum oxyfluoride). Generally, at least 50% of the fluorine atoms are bonded to an atom bearing an oxygen atom in an oxyfluoride material.

By "nanostructured" is meant a material in a form having at least one dimension that is, on average, at most 200 nanometers (e.g., nanosized particles). Thus, nanostructured materials refer to materials including, for example, nanoparticles as defined herein below; aggregates of nanoparticles; materials coated on particles, wherein the coatings have an average thickness of at most 200 nanometers; materials coated on aggregates of particles, wherein the coatings have an average thickness of at most 200 nanometers; materials infiltrated in porous structures having an average pore size of at most 200 nanometers; and combinations thereof. Porous structures include, for example, porous particles, porous aggregates of particles, porous coatings, and combinations thereof.

As used herein "nanoparticles" is used synonymously with "nanosized particles," and refers to particles having an average size of at most 200 nanometers. As used herein for a spherical particle, "size" refers to the diameter of the particle. As used herein for a non-spherical particle, "size" refers to the longest dimension of the particle. By "nanocluster" is meant an association of nanoparticles drawn together by relatively weak intermolecular forces that cause them to clump together, i.e. to aggregate. Typically, nanoclusters have an average size of at most 10 micrometers.

The term "ethylenically unsaturated compounds with acid functionality" is meant to include monomers, oligomers, and polymers having ethylenic unsaturation and acid and/or acid-precursor functionality. Acid-precursor functionalities include, for example, anhydrides, acid halides, and pyrophosphates.

By "dental compositions and dental articles" is meant to include orthodontic compositions (e.g., orthodontic adhesives) and orthodontic devices (e.g., orthodontic appliances such as retainers, night guards, brackets, buccal tubes, bands, cleats, buttons, lingual retainers, bite openers, positioners, and the like).

As used herein, a "(meth)acrylate" group is a shorthand term referring to both an acrylate group (i.e., CH₂=CHC(O)O-) and a methacrylate group (i.e., CH₂=C(CH₃)C(O)O-).

The recitation of numerical ranges by endpoints includes all numbers subsumed within that range (e.g. 1 to 5 includes 1, 1.5, 2, 2.75, 3, 3.80, 4, and 5).

As used in this specification and the appended claims, the singular forms "a", "an", and "the" include plural referents unless the content clearly dictates otherwise. Thus, for example, reference to a composition containing "a compound" includes a mixture of two or more compounds. As used in this specification and the appended claims, the term "or" is generally employed in its sense including "and/or" unless the content clearly dictates otherwise.

Unless otherwise indicated, all numbers expressing quantities of ingredients, measurement of properties such as contrast ratio and so forth used in the specification and claims are to be understood as being modified in all instances by the term "about." Accordingly, unless indicated to the contrary, the numerical parameters set forth in the foregoing specification and attached claims are approximations that can vary depending upon the desired properties sought to be obtained by those skilled in the art utilizing the teachings of the present invention. At the very least, and not as an attempt to limit the application of the doctrine of equivalents to the scope of the claims, each numerical parameter should at least be construed in light of the number of reported significant digits and by applying ordinary rounding techniques. Notwithstanding that the numerical ranges and parameters setting forth the broad scope of the invention are approximations, the numerical values set forth in the specific examples are reported as precisely as possible. Any numerical value, however, inherently contains certain errors necessarily resulting from the standard deviations found in their respective testing measurements.

### Brief Description of the Drawings

The invention may be more completely understood in consideration of the following detailed description of various embodiments of the invention in connection with the accompanying drawings, in which:
Fig. 1 is an SEM image of a broken specimen of a hardened composition as described below in Example 1. The image has a magnification of 200. The image shows a homogenous phase, in which no separation of any organic or inorganic components can be observed.
Fig. 2 is another SEM image of a broken specimen of a hardened composition as described below in Example 1. The image has a magnification of 1000. The image shows a homogenous phase, in which no separation of any organic or inorganic components can be observed.

While the invention is amenable to various modifications and alternative forms, specifics thereof have been shown by way of example in the drawings and will be described in detail. It should be understood, however, that the intention is not to limit the invention to the particular embodiments described. On the contrary, the intention is to cover all modifications, equivalents, and alternatives falling within the spirit and scope of the invention.

### Detailed Description

The present invention as defined in claim 1 is directed to dental compositions, specifically ionomer compositions, e.g., glass ionomer compositions, containing a filler component that has been surface-modified with a polyacid. These hardenable compositions typically further comprise an additional polyacid component that is not attached to the filler; a polymerizable component; an acid-reactive filler, such as FAS glass; and water. The incorporation into the composition of a filler that has been surface-modified with a polyacid provides for improved properties, including, for example, the ability to form a stable RMGI compositions without the need for HEMA, thereby eliminating the drawbacks associated with the use of HEMA in dental compositions (e.g., reduction in aesthetic properties).

### POLYMERIZABLE COMPONENT

As mentioned above, the hardenable dental compositions of the present invention include a polymerizable component. The polymerizable component is an ethylenically unsaturated compound with or without acid functionality.

The polymerizable component of the present invention can be part of a hardenable resin. These resins are generally thermosetting materials capable of being hardened to form a polymer network including, for example, acrylate-functional materials, methacrylate-functional materials, epoxy-functional materials, vinyl-functional materials, and mixtures thereof. Typically, the hardenable resin is made from one or more matrix-forming oligomer, monomer, polymer, or blend thereof.

In certain embodiments where the dental composition disclosed in the present application is a dental composite, polymerizable materials suitable for use include hardenable organic materials having sufficient strength, hydrolytic stability, and non-toxicity to render them suitable for use in the oral environment. Examples of such materials include acrylates, methacrylates, urethanes, carbamoylisocyanurates, epoxies, and mixtures and derivatives thereof.

One class of preferred hardenable materials includes materials having polymerizable components with free radically active functional groups. Examples of such materials include monomers having one or more ethylenically unsaturated groups, oligomers having one or more ethylenically unsaturated groups, polymers having one or more ethylenically unsaturated groups, and combinations thereof.

In the class of hardenable resins having free radically active functional groups, suitable polymerizable components for use in the invention contain at least one ethylenically unsaturated bond, and are capable of undergoing addition polymerization. Such free radically ethylenically unsaturated compounds include, for example, mono-, di- or poly-(meth)acrylates (i.e., acrylates and methacrylates) such as, methyl (meth)acrylate, ethyl acrylate, isopropyl methacrylate, n-hexyl acrylate, stearyl acrylate, allyl acrylate, glycerol triacrylate, ethyleneglycol diacrylate, diethyleneglycol diacrylate, triethyleneglycol dimethacrylate, 1,3-propanediol di(meth)acrylate, trimethylolpropane triacrylate, 1,2,4-butanetriol trimethacrylate, 1,4-cyclohexanediol diacrylate, pentaerythritol tetra(meth)acrylate, sorbitol hexacrylate, tetrahydrofurfuryl (meth)acrylate, bis[I-(2-acryloxy)]-p-ethoxyphenyldimethylmethane, bis[I-(3-acryloxy-2-hydroxy)]-p-propoxyphenyldimethylmethane, ethoxylated bisphenol A di(meth)acrylate, and trishydroxyethyl-isocyanurate trimethacrylate; (meth)acrylamides (i.e., acrylamides and methacrylamides) such as (meth)acrylamide, methylene bis-(meth)acrylamide, and diacetone (meth)acrylamide; urethane (meth)acrylates; the bis-(meth)acrylates of polyethylene glycols (preferably of molecular weight 200-500); copolymerizable mixtures of acrylated monomers such as those in U.S. Pat. No. 4,652, 274 (Boettcher et al.); acrylated oligomers such as those of U.S. Pat. No. 4,642,126 (Zador et al.); and vinyl compounds such as styrene, diallyl phthalate, divinyl succinate, divinyl adipate and divinyl phthalate. Other suitable free radically polymerizable compounds include siloxane-functional (meth)acrylates as disclosed, for example, in WO-00/38619 (Guggenberger et al.), WO-01/92271 (Weinmann et al.), WO-01/07444 (Guggenberger et al.), WO-00/42092 (Guggenberger et al.) and fluoropolymer-functional (meth)acrylates as disclosed, for example, in U.S. Pat. No. 5,076,844 (Fock et al.), U.S. Pat. No. 4,356,296 (Griffith et al.), EP-0 373 384 (Wagenknecht et al.), EP-0 201 031 (Reiners et al.), and EP-0 201 778 (Reiners et al.). Mixtures of two or more free radically polymerizable compounds can be used if desired.

The polymerizable component may also contain hydroxyl groups and free radically active functional groups in a single molecule. Examples of such materials include hydroxyalkyl (meth)acrylates, such as 2-hydroxyethyl (meth)acrylate and 2-hydroxypropyl (meth)acrylate; glycerol mono- or di-(meth)acrylate; trimethylolpropane mono- or di-(meth)acrylate; pentaerythritol mono-, di-, and tri-(meth)acrylate; sorbitol mono-, di-, tri-, tetra-, or penta-(meth)acrylate; and 2,2-bis[4-(2-hydroxy-3-methacryloxypropoxy)phenyl]propane (bisGMA). Suitable ethylenically unsaturated compounds are also available from a wide variety of commercial sources, such as Sigma-Aldrich, St. Louis, MO. Mixtures of ethylenically unsaturated compounds can be used if desired.

Typically, compositions of the present invention include at least 5% by weight, more typically at least 10% by weight, and most typically at least 15% by weight ethylenically unsaturated compounds, based on the total weight of the unfilled composition. Typically, compositions of the present invention include at most 95% by weight, more typically at most 90% by weight, and most typically at most 80% by weight ethylenically unsaturated compounds, based on the total weight of the unfilled composition.

Typically, compositions of the present invention include at least 5% by weight (wt-%), more typically at least 10% by weight, and most typically at least 15% by weight ethylenically unsaturated compounds without acid functionality, based on the total weight of the unfilled composition. Typically, compositions of the present invention include at most 95% by weight, more typically at most 90% by weight, and most typically at most 80% by weight ethylenically unsaturated compounds without acid functionality, based on the total weight of the unfilled composition.

### POLYMERIZABLE COMPONENT WITH ACID FUNCTIONALITY

When present, the polymerizable component optionally comprises an ethylenically unsaturated compound with acid functionality. Preferably, the acid functionality includes an oxyacid (i.e., an oxygen-containing acid) of carbon, sulfur, phosphorous, or boron.

Such compounds include, for example, α,β-unsaturated acidic compounds such as glycerol phosphate monomethacrylates, glycerol phosphate dimethacrylates, hydroxyethyl methacrylate phosphates, citric acid di- or trimethacrylates, poly(meth)acrylated oligomaleic acid, poly(meth)acrylated polymaleic acid, poly(meth)acrylated poly(meth)acrylic acid, poly(meth)acrylated polycarboxyl-polyphosphonic acid, poly(meth)acrylated polychlorophosphoric acid, poly(meth)acrylated polysulfonic acid, poly(meth)acrylated polyboric acid, and the like, may be used as components in the hardenable resin system.

Certain of these compounds are obtained, for example, as reaction products between isocyanatoalkyl (meth)acrylates and carboxylic acids. Additional compounds of this type having both acid-functional and ethylenically unsaturated components are described in U.S. Pat. Nos. 4,872,936 (Engelbrecht) and 5,130,347 (Mitra). A wide variety of such compounds containing both the ethylenically unsaturated and acid moieties can be used. Mixtures of such compounds can be used if desired.

Additional ethylenically unsaturated compounds with acid functionality include, for example, polymerizable bisphosphonic acids as disclosed for example, in U.S.S.N. 10/729,497; AA:ITA:IEM (copolymer of acrylic acid:itaconic acid with pendent methacrylate made by reacting AA:ITA copolymer with sufficient 2-isocyanatoethyl methacrylate to convert a portion of the acid groups of the copolymer to pendent methacrylate groups as described, for example, in Example 11 of U.S. Pat. No. 5,130,347 (Mitra)); and those recited in U.S. Pat. Nos. 4,259,075 (Yamauchi et al.), 4,499,251 (Omura et al.), 4,537,940 (Omura et al.), 4,539,382 (Omura et al.), 5,530,038 (Yamamoto et al.), 6,458,868 (Okada et al.), and European Pat. Application Publication Nos. EP 712,622 (Tokuyama Corp.) and EP 1,051,961 (Kuraray Co., Ltd.).

When ethylenically unsaturated compounds with acid functionality are present, the compositions of the present invention typically include at least 1 % by weight, more typically at least 3% by weight, and most typically at least 5% by weight ethylenically unsaturated compounds with acid functionality, based on the total weight of the unfilled composition. Typically, compositions of the present invention include at most 50% by weight, more typically at most 40% by weight, and most typically at most 30% by weight ethylenically unsaturated compounds with acid functionality, based on the total weight of the unfilled composition.

Partial or complete hardening of the composition may occur through an acid-reactive filler/polyacid reaction (i.e. an acid/base reaction). In certain embodiments, the composition also contains a photoinitiator system that upon irradiation with actinic radiation initiates the polymerization (or hardening) of the composition. Such photopolymerizable compositions can be free radically polymerizable.

### INITIATION SYSTEMS

For free radical polymerization (e.g., hardening), an initiation system can be selected from systems that initiate polymerization via radiation, heat, or redox/auto-cure chemical reaction. A class of initiators capable of initiating polymerization of free radically active functional groups includes free radical-generating photoinitiators, optionally combined with a photosensitizer or accelerator. Such initiators typically can be capable of generating free radicals for addition polymerization upon exposure to light energy having a wavelength between 200 and 800 nm.

Suitable photoinitiators (i.e., photoinitiator systems that include one or more compounds) for polymerizing free radically photopolymerizable compositions include binary and ternary systems. Typical ternary photoinitiators include an iodonium salt, a photosensitizer, and an electron donor compound as described in U.S. Pat. No. 5,545,676 (Palazzotto et al.). Preferred iodonium salts are the diaryl iodonium salts, e.g., diphenyliodonium chloride, diphenyliodonium hexafluorophosphate, diphenyliodonium tetrafluoroborate, and tolylcumyliodonium tetrakis(pentafluorophenyl) borate. Preferred photosensitizers are monoketones and diketones that absorb some light within a range of about 400 nm to 520 nm (preferably, 450 nm to 500 nm). More preferred compounds are alpha diketones that have some light absorption within a range of 400 nm to 520 nm (even more preferably, 450 to 500 nm). Preferred compounds are camphorquinone, benzil, furil, 3,3,6,6-tetramethylcyclohexanedione, phenanthraquinone, 1-phenyl-1,2-propanedione and other 1-aryl-2-alkyl-1,2-ethanediones, and cyclic alpha diketones. Most preferred is camphorquinone. Preferred electron donor compounds include substituted amines, e.g., ethyl dimethylaminobenzoate. Other suitable ternary photoinitiator systems useful for photopolymerizing cationically polymerizable resins are described, for example, in U.S. Pat. Publication No. 2003/0166737 (Dede et al.) and International Publication No. WO 2005/051332 (Oxman et al.).

Other suitable photoinitiators for polymerizing free radically photopolymerizable compositions include the class of phosphine oxides that typically have a functional wavelength range of 380 nm to 1200 nm. Preferred phosphine oxide free radical initiators with a functional wavelength range of 380 nm to 450 nm are acyl and bisacyl phosphine oxides such as those described in U.S. Pat. Nos. 4,298,738 (Lechtken et al.), 4,324,744 (Lechtken et al.), 4,385,109 (Lechtken et al.), 4,710,523 (Lechtken et al.), and 4,737,593 (Ellrich et al.), 6,251,963 (Kohler et al.); and EP Application No. 0 173 567 A2 (Ying).

Commercially available phosphine oxide photoinitiators capable of free-radical initiation when irradiated at wavelength ranges of greater than 380 nm to 450 nm include, for example, bis(2,4,6-trimethylbenzoyl)phenyl phosphine oxide available under the trade designation IRGACURE 819 from Ciba Specialty Chemicals, Tarrytown, NY; bis(2,6-dimethoxybenzoyl)-(2,4,4-trimethylpentyl) phosphine oxide available under the trade designation CGI 403 from Ciba Specialty Chemicals; a 25:75 mixture, by weight, of bis(2,6-dimethoxybenzoyl)-2,4,4-trimethylpentyl phosphine oxide and 2-hydroxy-2-methyl-1-phenylpropan-1-one available under the trade designation IRGACURE 1700 from Ciba Specialty Chemicals; a 1:1 mixture, by weight, of bis(2,4,6-trimethylbenzoyl)phenyl phosphine oxide and 2-hydroxy-2-methyl-1-phenylpropane-1-one available under the trade designation DAROCUR 4265 from Ciba Specialty Chemicals; and ethyl 2,4,6-trimethylbenzylphenyl phosphinate available under the trade designation LUCIRIN LR8893X from BASF Corp., Charlotte, NC.

Typically, the phosphine oxide initiator is present in the photopolymerizable composition in catalytically effective amounts, such as from 0.1 % by weight to 5% by weight, based on the total weight of the composition.

Tertiary amine reducing agents may be used in combination with an acylphosphine oxide. Illustrative tertiary amines useful in the invention include ethyl 4-(N,N-dimethylamino)benzoate and N,N-dimethylaminoethyl methacrylate. When present, the amine reducing agent is present in the photopolymerizable composition in an amount from 0.1 % by weight to 5% by weight, based on the total weight of the composition. Useful amounts of other initiators are well known to those of skill in the art.

Another free-radical initiator system that can alternatively be used in the dental materials of the invention includes the class of ionic dye-counterion complex initiators including a borate anion and a complementary cationic dye. Borate salt photoinitiators are described, for example, in U. S. Pat. Nos. 4,772,530 (Gottschalk et al.), 4,954,414 (Adair et al.), 4,874,450 (Gottschalk), 5,055,372 (Shanklin et al.), and 5,057,393 (Shanklin et al.).

The hardenable resins of the present invention can include redox cure systems that include a polymerizable component (e.g., an ethylenically unsaturated polymerizable component) and redox agents that include an oxidizing agent and a reducing agent. Suitable polymerizable components and redox agents that are useful in the present invention are described in U.S. Pat. Publication No. 2003/0166740 (Mitra et al.) and U.S. Pat. Publication No. 2003/0195273 (Mitra et al.).

The reducing and oxidizing agents should react with or otherwise cooperate with one another to produce free-radicals capable of initiating polymerization of the resin system (e.g., the ethylenically unsaturated component). This type of cure is a dark reaction, that is, it is not dependent on the presence of light and can proceed in the absence of light. The reducing and oxidizing agents are preferably sufficiently shelf-stable and free of undesirable colorization to permit their storage and use under typical dental conditions. They should be sufficiently miscible with the resin system (and preferably water-soluble) to permit ready dissolution in (and discourage separation from) the other components of the polymerizable composition.

Useful reducing agents include, for example, ascorbic acid, ascorbic acid derivatives, and metal complexed ascorbic acid compounds as described in U.S. Pat. No. 5,501,727 (Wang et al.); amines, especially tertiary amines, such as 4-tert-butyl dimethylaniline; aromatic sulfinic salts, such as p-toluenesulfinic salts and benzenesulfinic salts; thioureas, such as 1-ethyl-2-thiourea, tetraethyl thiourea, tetramethyl thiourea, 1,1-dibutyl thiourea, and 1,3-dibutyl thiourea; and mixtures thereof. Other secondary reducing agents may include cobalt (II) chloride, ferrous chloride, ferrous sulfate, hydrazine, hydroxylamine (depending on the choice of oxidizing agent), salts of a dithionite or sulfite anion, and combinations thereof. Preferably, the reducing agent is an amine.

Suitable oxidizing agents will also be familiar to those skilled in the art, and include, for example, persulfuric acid and salts thereof, such as sodium, potassium, ammonium, cesium, and alkyl ammonium salts. Additional oxidizing agents include, for example, peroxides such as benzoyl peroxides, hydroperoxides such as cumyl hydroperoxide, t-butyl hydroperoxide, and amyl hydroperoxide, as well as salts of transition metals such as cobalt (III) chloride and ferric chloride, cerium (IV) sulfate, perboric acid and salts thereof, permanganic acid and salts thereof, perphosphoric acid and salts thereof, and combinations thereof.

It may be desirable to use more than one oxidizing agent or more than one reducing agent. Small quantities of transition metal compounds may also be added to accelerate the rate of redox cure. In some embodiments it may be preferred to include a secondary ionic salt to enhance the stability of the hardenable composition as described, for example, in U.S. Pat. Publication No. 2003/0195273 (Mitra et al.).

The reducing and oxidizing agents are present in amounts sufficient to permit an adequate free-radical reaction rate. This can be evaluated by combining all of the ingredients of the hardenable composition except for the filler, and observing whether or not a hardened mass is obtained.

Preferably, the reducing agent is present in an amount of at least 0.01% by weight, and more preferably at least 0.10% by weight, based on the total weight (including water) of the components of the hardenable composition. Preferably, the reducing agent is present in an amount of no greater than 10% by weight, and more preferably no greater than 5% by weight, based on the total weight (including water) of the components of the polymerizable composition.

Preferably, the oxidizing agent is present in an amount of at least 0.01% by weight, and more preferably at least 0.10% by weight, based on the total weight (including water) of the components of the polymerizable composition. Preferably, the oxidizing agent is present in an amount of no greater than 10% by weight, and more preferably no greater than 5% by weight, based on the total weight (including water) of the components of the hardenable composition.

The reducing or oxidizing agents can be microencapsulated as described, for example, in U.S. Pat. No. 5,154,762 (Mitra et al.). This will generally enhance shelf stability of the polymerizable composition, and if necessary permit packaging the reducing and oxidizing agents together. For example, through appropriate selection of an encapsulant, the oxidizing and reducing agents can be combined with an acid-functional component and optional filler and kept in a storage-stable state. Likewise, through appropriate selection of a water-insoluble encapsulant, the reducing and oxidizing agents can be combined with an FAS glass and water and maintained in a storage-stable state.

In a further alternative, heat may be used to initiate the hardening, or polymerization, of free radically active groups. Examples of heat sources suitable for the dental materials of the invention include inductive, convective, and radiant. Thermal sources should be capable of generating temperatures of at least 40°C and at most 150°C under normal conditions or at elevated pressure. This procedure is preferred for initiating polymerization of materials occurring outside of the oral environment.

Yet another alternative class of initiators capable of initiating polymerization of free radically active functional groups in the hardenable resin are those that include free radical-generating thermal initiators. Examples include peroxides (e.g., benzoyl peroxide and lauryl peroxide) and azo compounds (e.g., 2,2-azobisisobutyronitrile (AIBN)).

Photoinitiator compounds are preferably provided in dental compositions disclosed in the present application in an amount effective to initiate or enhance the rate of cure or hardening of the resin system. Useful photopolymerizable compositions are prepared by simply admixing, under safe light conditions, the components as described above. Suitable inert solvents may be used, if desired, when preparing this mixture. Any solvent that does not react appreciably with the components of the inventive compositions may be used. Examples of suitable solvents include, for example, acetone, dichloromethane, and acetonitrile.

### POLYACID

Compositions of the present invention include at least one polyacid, which may be a non-curable or non-polymerizable polyacid, or a curable or polymerizable polyacid (e.g., a resin-modified polyacid). Typically, the polyacid is a polymer having a plurality of acidic repeating units and/or a plurality of polymerizable groups. In alternative embodiments, the polyacid may be substantially free of polymerizable groups. The polyacid need not be entirely water soluble, but typically it is at least sufficiently water-miscible so that it does not undergo substantial sedimentation when combined with other aqueous components. Suitable polyacids are listed in U.S. Pat. No. 4,209,434 (Wilson et al.), column 2, line 62, to column 3, line 6. The polyacid should have a molecular weight sufficient to provide good storage, handling, and mixing properties. A typical weight average molecular weight is 5,000 to 100,000, evaluated against a polystyrene standard or a polyacrylic acid sodium salt standard using gel permeation chromatography.

In one embodiment, the polyacid is a curable or polymerizable resin; that is, it contains at least one ethylenically unsaturated group. Suitable ethylenically unsaturated polyacids are described in U.S. Pat. No. 4,872,936 (Engelbrecht), e.g., at columns 3 and 4, and EP 323 120 B1 (Mitra), e.g., at page 3, line 55 to page 5, line 8. Typically, the numbers of acidic groups and ethylenically unsaturated groups are adjusted to provide an appropriate balance of properties in the dental composition. Polyacids in which 10% to 70% of the acidic groups have been replaced with ethylenically unsaturated groups are preferred.

In other embodiments, the polyacid is hardenable in the presence of, for example, an acid-reactive filler and water, but does not contain ethylenically unsaturated groups; that is, it is an oligomer or polymer of an unsaturated acid. Typically, the unsaturated acid is an oxyacid (i.e., an oxygen containing acid) of carbon, sulfur, phosphorous, or boron. More typically, it is an oxyacid of carbon. Such polyacids include, for example, polyalkenoic acids such as homopolymers and copolymers of unsaturated mono-, di-, or tricarboxylic acids. Polyalkenoic acids can be prepared by the homopolymerization and copolymerization of unsaturated aliphatic carboxylic acids, e.g., acrylic acid, 2-choloracrylic acid, 3-choloracrylic acid, 2-bromoacrylic acid, 3-bromoacrylic acid, methacrylic acid, itaconic acid, maleic acid, glutaconic acid, aconitic acid, citraconic acid, mesaconic acid, fumaric acid, and tiglic acid. Suitable monomers that can be copolymerized with the unsaturated aliphatic carboxylic acids include, for example, unsaturated aliphatic compounds such as acrylamide, acrylonitrile, vinyl chloride, allyl chloride, vinyl acetate, and 2-hydroxyethyl methacrylate. Ter- and higher polymers may be used if desired. Particularly preferred are the homopolymers and copolymers of acrylic acid. The polyalkenoic acid should be substantially free of unpolymerized monomers. In another embodiment, the polyacid is a copolymer of acrylic acid and α-β unsaturated carboxylic acids, e.g. maleic or itaconic acid. In yet another embodiment, polymers or copolymers bearing phosphonic acid, phosphoric acid or sulfonic acids can be used.

The amount of polyacid should be sufficient to react with the acid-reactive filler and to provide an ionomer composition with desirable hardening properties. Typically, the polyacid represents at least 1 wt-%, more typically at least 3 wt-%, and most typically at least 5 wt-%, based on the total weight of the unfilled composition. Typically, the polyacid represents at most 90 wt-%, more typically at most 60 wt-%, and most typically at most 30 wt-%, based on the total weight of the unfilled composition.

### FILLER SURFACE-MODIFIED WITH A POLYACID

In addition to the polyacid component, the compositions of the present invention also include a filler that has been surface-modified with a polyacid, i.e. a filler to which a polyacid has been attached. The typical molecule weight of the polyacid is in the range of 1000 to 500,000 with 5,000 to 150,000 being generally preferred. The polyacid may be a statistical copolymer or a blockcopolymer or an alternating copolymer. It even may be a linear polymer or a branched polymer, for example a star formed polymer. Typically, the surface of the filler is first modified with a linking group and subsequently the polyacid is connected to this linking group. Functionalized alkoxysilanes can be attached to the surface of a variety of inorganic fillers including aluminum hydroxide, cristoballit, glass including glass fibers, kaoline, precipitated or pyrogenic silica quartz, quartz glass, and wollastonit. Other suitable inorganic oxides may be used, as well as mica, silicates (e.g., feldspar), magnesium hydroxide, talc, nickel oxide, and the like. In addition, FAS glasses with low metal content, organic or polymeric fillers containing C-OH groups, groups on the surface are suitable fillers for use in the composition of the invention.

### ACID-REACTIVE FILLERS

Suitable acid-reactive fillers include metal oxides, glasses, and metal salts. Typical metal oxides include barium oxide, calcium oxide, magnesium oxide, and zinc oxide. Typical glasses include borate glasses, phosphate glasses, and fluoroaluminosilicate ("FAS") glasses. FAS glasses are particularly preferred. The FAS glass typically contains sufficient elutable cations so that a hardened dental composition will form when the glass is mixed with the components of the hardenable composition. The glass also typically contains sufficient elutable fluoride ions so that the hardened composition will have cariostatic properties. The glass can be made from a melt containing fluoride, alumina, and other glass-forming ingredients using techniques familiar to those skilled in the FAS glassmaking art. The FAS glass typically is in the form of particles that are sufficiently finely divided so that they can conveniently be mixed with the other cement components and will perform well when the resulting mixture is used in the mouth.

Generally, the average particle size (typically, diameter) for the FAS glass is no greater than about 12 micrometers, typically no greater than 10 micrometers, and more typically no greater than 5 micrometers as measured using, for example, a sedimentation analyzer. Suitable FAS glasses will be familiar to those skilled in the art, and are available from a wide variety of commercial sources, and many are found in currently available glass ionomer cements such as those commercially available under the trade designations VITREMER, VITREBOND, RELY X LUTING CEMENT, RELY X LUTING PLUS CEMENT, PHOTAC-FIL QUICK, KETAC-MOLAR, and KETAC-FIL PLUS (3M ESPE Dental Products, St. Paul, MN), FUJI II LC and FUJI IX (G-C Dental Industrial Corp., Tokyo, Japan) and CHEMFIL Superior (Dentsply International, York, PA). Mixtures of fillers can be used if desired.

The FAS glass can optionally be subjected to a surface treatment. Suitable surface treatments include, but are not limited to, acid washing (e.g., treatment with a phosphoric acid), treatment with a phosphate, treatment with a chelating agent such as tartaric acid, and treatment with a silane or an acidic or basic silanol solution. Desirably the pH of the treating solution or the treated glass is adjusted to neutral or near-neutral, as this can increase storage stability of the hardenable composition.

In another embodiment, the acid-reactive filler comprises a non-fused oxyfluoride material. The oxyfluoride material may include a trivalent metal, oxygen, fluorine, and an alkaline earth metal. Preferably the trivalent metal is aluminum, lanthanum, yttrium or combinations thereof. More preferably the trivalent metal is aluminum. Preferably the alkaline earth metal is strontium, calcium, barium, or combinations thereof. In some embodiments of the present invention, the oxyfluoride material may further include silicon and/or heavy metal (e.g., zirconium, lanthanum, niobium, yttrium, or tantalum), or more specifically, oxides, fluorides and/or oxyfluorides thereof.

In some embodiments of the present invention, at least a portion of the oxyfluoride material is nanostructured. Such nanostructured materials include the oxyfluoride material in the form of, for example, nanoparticles, coatings on particles, coatings on aggregates of particles, infiltrate in a porous structure, and combinations thereof. Preferably at least 90% by weight, more preferably at least 95% by weight, and most preferably at least 98% by weight of the oxyfluoride material is nanostructured.

A description of suitable oxyfluoride materials and their use in dental compositions is provided in U.S. Patent Application Serial No. 10/847,805 (Budd et al.) The amount of acid-reactive filler should be sufficient to provide an ionomer composition having desirable mixing and handling properties before hardening and good physical and optical properties after hardening. Generally, the reactive filler represents less than about 85% of the total weight of the composition. Typically, the acid-reactive filler represents at least 10 wt-%, and more typically at least 20 wt-%, based on the total weight of the composition. Typically, the acid-reactive filler represents at most 75 wt-%, and more typically at most 50 wt-%, based on the total weight of the composition.

### OTHER FILLERS

In addition to the acid-reactive filler, the compositions of the present invention can also optionally include one or more other fillers. Such fillers may be selected from one or more of a wide variety of materials suitable for the use in dental and/or orthodontic compositions.

The other filler can be an inorganic material. It can also be a crosslinked organic material that is insoluble in the resin component of the composition, and is optionally filled with inorganic filler. The filler should in any event be nontoxic and suitable for use in the mouth. The filler can be radiopaque or radiolucent. The filler typically is substantially insoluble in water.

Examples of suitable inorganic fillers are naturally occurring or synthetic materials including, but not limited to: quartz; nitrides (e.g., silicon nitride); glasses derived from, for example, Zr, Sr, Ce, Sb, Sn, Ba, Zn, and Al; feldspar; borosilicate glass; kaolin; talc; titania; low Mohs hardness fillers such as those described in U.S. Pat. No. 4,695,251 (Randklev); and silica particles (e.g., submicron pyrogenic silicas such as those available under the trade designations AEROSIL, including "OX 50," "130," "150" and "200" silicas from Degussa AG, Hanau, Germany and CAB-O-SIL M5 silica from Cabot Corp., Tuscola, IL). Examples of suitable organic filler particles include filled or unfilled pulverized polycarbonates, polyepoxides, and the like.

Suitable non-acid-reactive filler particles are quartz, submicron silica, and non-vitreous microparticles of the type described in U.S. Pat. No. 4,503,169 (Randklev). Mixtures of these non-acid-reactive fillers are also contemplated, as well as combination fillers made from organic and inorganic materials.

The surface of the filler particles can also be treated with a coupling agent in order to enhance the bond between the filler and the resin. The use of suitable coupling agents include gamma-methacryloxypropyltrimethoxysilane, gamma-mercaptopropyltriethoxysilane, gamma-aminopropyltrimethoxysilane, and the like. Examples of useful silane coupling agents are those available from Crompton Corporation, Naugatuck, CT, as SILQUEST A-174 and SILQUEST A-1230.

For some embodiments of the present invention that include other fillers (e.g., dental restorative compositions), the compositions may include at least 1% by weight, more preferably at least 2% by weight, and most preferably at least 5% by weight other filler, based on the total weight of the composition. For such embodiments, compositions of the present invention preferably include at most 40% by weight, more preferably at most 20% by weight, and most preferably at most 15% by weight other filler, based on the total weight of the composition.

The composition of the invention may optionally contain one or more nanofillers which may be either acid reactive or non-acid reactive. In addition or as an alternative, the filler that has been surface modified with polyacid may optionally be a nanofiller.

Such nanofillers typically have an average particle size of at most 200 nanometers and more typically at most 100 nanometers. Such nanofillers typically have an average particle size of at least 2 nanometers and more typically at least 5 nanometers. Typically, the nanofiller comprises nanoparticles selected from silica; zirconia; oxides of titanium, aluminum, cerium, tin, yttrium, strontium, barium, lanthanum, zinc, ytterbium, bismuth, iron, and antimony; and combinations thereof. More typically, the nanofiller comprises nanoparticles selected from silica; zirconia; oxides of titanium; and combinations thereof. In some embodiments, the nanofiller is in the form of nanoclusters, typically at least 80 percent by weight nanoclusters. More typically the nanoclusters include silica clusters, silica-zirconia clusters, and combinations thereof. In other embodiments, the nanofiller is in the form of a combination of nanoparticles and nanoclusters. Often a portion of the surface of the nanofiller is silane treated or otherwise chemically treated to provide one or more desired physical properties.

Suitable nanofillers are disclosed in U.S. Pat. Nos. 6,387,981 (Zhang et al.) and 6,572,693 (Wu et al.) as well as International Publication Nos. WO 01/30305 (Zhang et al.), WO 01/30306 (Windisch et al.), WO 01/30307 (Zhang et al.), and WO 03/063804 (Wu et al.). Filler components described in these references include nanosized silica particles, nanosized metal oxide particles, and combinations thereof. Nanofillers are also described in U.S. Patent Application Serial Nos. 10/847,781 (Kangas et al.); 10/847,782 (Kolb et al.); and 10/847,803 (Craig et al.). Typically, the nanofillers are non-pyrogenic fillers, however pyrogenic fillers can be added as optional additives to the dental compositions.

The acid-reactive, non-fused oxyfluoride materials described above that are at least partially nanostructured can be used as nanofillers in the present invention.
The amount of nanofiller should be sufficient to provide an ionomer composition having desirable mixing and handling properties before hardening and good physical and optical properties after hardening. Typically, the nanofiller represents at least 0.1 wt-%, more typically at least 10 wt-%, and most typically at least 20 wt-% based on the total weight of the composition. Typically, the nanofiller represents at most 80 wt-%, more typically at most 70 wt-%, and most typically at most 60 wt-%, based on the total weight of the composition.

### WATER

The compositions of the invention contain water. The water can be distilled, deionized, or plain tap water. Typically, deionized water is used.

The amount of water should be sufficient to provide adequate handling and mixing properties and to permit the transport of ions, particularly in the filler-acid reaction. Preferably, water represents at least 2 wt-%, and more preferably at least 5 wt-%, of the total weight of ingredients used to form the composition. Preferably, water represents no greater than 90 wt-%, and more preferably no greater than 80 wt-%, of the total weight of ingredients used to form the composition.

### OTHER ADDITIVES

Optionally, the hardenable compositions may contain other solvents, cosolvents (e.g., alcohols) or diluents. If desired, the hardenable composition of the invention can contain additives such as indicators, dyes, pigments, inhibitors, accelerators, viscosity modifiers, wetting agents, tartaric acid, chelating agents, surfactants, buffering agents, stabilizers, and other similar ingredients that will be apparent to those skilled in the art. Additionally, medicaments or other therapeutic substances can be optionally added to the dental compositions. Examples include, but are not limited to, fluoride sources, whitening agents, anticaries agents (e.g., xylitol), remineralizing agents (e.g., calcium phosphate compounds), enzymes, breath fresheners, anesthetics, clotting agents, acid neutralizers, chemotherapeutic agents, immune response modifiers, thixotropes, polyols, anti-inflammatory agents, antimicrobial agents, antifungal agents, agents for treating xerostomia, desensitizers, and the like, of the type often used in dental compositions. Combination of any of the above additives may also be employed. The selection and amount of any one such additive can be selected by one of skill in the art to accomplish the desired result without undue experimentation.

### PREPARATION AND USE OF THE COMPOSITIONS

The hardenable dental compositions of the present invention can be prepared by combining all the various components using conventional mixing techniques. As discussed above, the compositions may be partially or fully hardened by an ionic reaction between an acid-reactive filler and a polyacid. Optionally, the compositions may contain a polymerizable component and a photoinitiator and be hardened by photoinitiation, or may be partially or fully hardened by chemical polymerization such as a redox cure system in which the composition contains a free-radical initiator system, e.g., including an oxidizing agent and a reducing agent. Alternatively, the hardenable composition may contain different initiator systems, such that the composition can be both a photopolymerizable and a chemically polymerizable composition, as well as an ionically hardenable composition.

The hardenable compositions of the invention can be supplied in a variety of forms including one-part systems and multi-part systems, e.g., two-part powder/liquid, paste/liquid, paste/powder and paste/paste systems. Other forms employing multi-part combinations (i.e., combinations of two or more parts), each of which is in the form of a powder, liquid, gel, or paste are also possible. The various components of the composition may be divided up into separate parts in whatever manner is desired; however, the polyacid, acid-reactive filler and water generally would not all be present in the same part, although any two of these may be grouped together in the same part along with any combination of other components. Furthermore, in a redox multi-part system, one part typically contains the oxidizing agent and another part typically contains the reducing agent. However, the reducing agent and oxidizing agent could be combined in the same part of the system if the components are kept separated, for example, through use of microencapsulation.

In one embodiment, the composition of the present invention is provided as a two-part, paste-paste system. The first part, Paste A, typically contains a light cure catalyst, an FAS glass, an ethylenically unsaturated component(s), and optionally pigments, rheology modifiers, and fluoride sources. The second part, Paste B, typically contains a polyacid, a filler surface-treated with a polyacid, a filler surface treated with silane, an optional ethylenically unsaturated component, water, and optionally pigments, rheology modifiers, and fluoride sources. Typically, neither Paste A nor Paste B contains HEMA. This combination of ingredients in Paste A and Paste B generally provides a stable RMGI composition with primerless adhesion to dentin and enamel. Such compositions are especially useful for bulk filling of tooth restorations by a convenient, one-step, easy mix direct restoration method.

In some embodiments, two-part dental compositions of the present invention can be provided in a dual barrel syringe having a first barrel and a second barrel, wherein the part A resides in the first barrel and the part B resides in the second barrel. In other embodiments, two-part dental compositions of the present invention can be provided in a unit-dose capsule. In some embodiments, each part of a multi-part dental system can be mixed together using a static mixer.

The components of the hardenable composition can be included in a kit, where the contents of the composition are packaged to allow for storage of the components until they are needed.

When used as a dental composition, the components of the hardenable compositions can be mixed and clinically applied using conventional techniques. A curing light is generally required for the initiation of photopolymerizable compositions. The compositions can be in the form of composites or restoratives that adhere very well to dentin and/or enamel. Optionally, a primer layer can be used on the tooth tissue on which the hardenable composition is used. The compositions, e.g., containing a FAS glass or other fluoride-releasing material, can also provide very good long-term fluoride release. Some embodiments of the invention may provide glass ionomer cements or adhesives that can be cured in bulk without the application of light or other external curing energy, do not require a pre-treatment, have improved physical properties including improved flexural strength, and have high fluoride release for cariostatic effect.

The hardenable dental compositions of the invention are particularly well adapted for use in the form of a wide variety of dental materials. They can be used in prosthodontic cements, which are typically filled compositions (preferably containing greater than about 25 wt-% filler and up to about 60 wt-% filler). They can also be used in restoratives, which include composites which are typically filled compositions (preferably containing greater than about 10 wt-% filler and up to about 85 wt-% filler) that are polymerized after being disposed adjacent to a tooth, such as filling materials. They can also be used in prostheses that are shaped and hardened for final use (e.g., as a crown, bridge, veneer, inlay, onlay, or the like), before being disposed adjacent to a tooth. Such preformed articles can be ground or otherwise formed into a custom-fitted shape by the dentist or other user. Although the hardenable dental composition can be any of a wide variety of materials preferably, the composition is not a surface pre-treatment material (e.g., etchant, primer, bonding agent). Rather, preferably, the hardenable dental composition is a restorative (e.g., composite, filling material or prosthesis), cement, sealant, coating, or orthodontic adhesive.

Features and advantages of this invention are further illustrated by the following examples, which are in no way intended to be limiting thereof. The particular materials and amounts thereof recited in these examples, as well as other conditions and details, should not be construed to unduly limit this invention. Unless otherwise indicated, all parts and percentages are on a weight basis, all water is deionized water, and all molecular weights are weight average molecular weight.

### EXAMPLES

### Test Methods

### SEM (Scanning Electron Microscope) Test Method

SEM was used to investigate fractured test samples of hardened compositions. Measurements were made with a Hitachi S 510 Scanning Electron Microscope. Samples investigated were broken specimens (25-mm length x 2-mm width x 2-mm thickness). Before scanning, surfaces were sputtered for 180 s with a gold-palladium alloy under a current of 50 mA. Specimens were scanned under low pressure (2 x 10⁻⁴ mbar) and an acceleration voltage of 25 kV was used.

### Flexural Strength (FS) Test Method

Flexural strength was determined according to ISO Standard 9917-2 (1998) using a Zwick universal tester (Zwick GmbH & Co. KG, Ulm, Germany) operated at a crosshead speed of 1 millimeter per minute (mm/min). Results were reported as the average of 6 replicates. Specimens were prepared by mixing test sample pastes with a spatula, transferring the mixed pastes into rectangular-shaped molds (25-mm length x 2-mm width x 2-mm thickness, and light-curing for 40 seconds using an ELIPAR TRILIGHT curing light (3M ESPE, Seefeld, Germany).

### Adhesion to Dentin Test Method

Adhesion tests were carried out according to the following test procedure using bovine teeth. For each test, five bovine teeth deep frozen following extraction were thawed, cleaned to remove the remaining gum, and separated from the roots by sawing with a diamond saw. The remaining pulp was removed with the aid of a pulp needle and the teeth were then rinsed with water. Planar dentin was obtained by labial sanding of the teeth on a water-cooled diamond sanding disk and finally treated with a fine silicone carbide sandpaper. The teeth were then embedded in silicone in such a way that the sanded-off surface, which was kept moistened, pointed upward. Then a mold consisting of a small wax plate, which had a round cutout of 6 mm in diameter (test area) and a height of 2 mm was put on each tooth. This test area was filled in a planar fashion with the test sample. Immediately after filling the mold, the sample was light-cured for 40 seconds by using a ELIPAR TRILIGHT curing light (3M ESPE). After curing, the small wax plate was removed and a screw bonded adhesively to the hardened sample at a right angle to the surface of the tooth. The screw was subsequently used to attach the system to the set-up of the testing apparatus. The teeth were stored at 36° C and > 95 % relative humidity for 24 hours. The adhesion was measured in a take-off test on a Zwick UPM 1455 Tester (Zwick GmbH & Co. KG) with a take-off rate of 1 mm/min. The adhesion was calculated in units of MPa and reported as an average of 5 replicates.

Unless otherwise stated, adhesion tests were conducted without any pretreatment in the form of a conditioner like GC Fuji Conditioner (GC Corp., Japan), a primer like VITRIMER Primer (3M ESPE), or a bonding system. It is state of the art that for conventional glass ionomer restoratives, adhesion is improved by using a conditioner. Similarly, for certain resin-modified glass ionomer restoratives, adhesion is achieved by using a primer. When used, the conditioner or primer products were used as stated in the manufacturer's instructions for use.

### Abbreviations, Descriptions, and Sources of Materials

| **Abbreviation** | **Description and Source of Material** |
|---|---|
| TPO-L | Lucirin TPO-L Photoinitiator (BASF AG, Germany) |
| UDMA | Diurethane dimethacrylate (CAS No. 41137-60-4), commercially available as Rohamere 6661-0 (Rohm Tech, Inc., Malden, MA) |
| Ebecryl 168 | Methacrylated acidic compound (Monomer) (UCB-Radcure Specialties, Brussels, Belgium) |
| DMF | N,N-Dimethylformamide, Merck, Rahway, NJ |
| QUARTZ FILLER | Ground Quartz Fluor; average particle size 0.9 µm (Quarzwerke, Frechen, Germany) |
| APS | 3-Aminopropyltriethoxysilane (ABCR GmbH KG, Karlsruhe, Germany) |
| POLYACID A | Poly(acrylic) acid spray-dried powder prepared as described below. |
| Filler A | Fluoroaluminosilicate (FAS) glass filler available as KETAC Molar Hand Mix powder. (3M ESPE) |
| Filler B | Surface modified quartz bearing poly acid. QUARTZ FILLER that has been surface-modified with POLYACID A according to the general process disclosed in the publication: K. Suzuki, S. Siddiqui, C. Chappel, J. A. Siddiqui, M. Ottenbrite; Polym. Adv. Technol. 2000, 11, 92 - 97. The specific preparation is described below. |
| Filler C | QUARTZ FILLER was silanized with 5% 3-Methacryloxypropyltrimethoxysilane (CAS: 2530-85-0) (Wacker Chemie GmbH, Munich, Germany) |
| Phosphoric Acid | Aqueous solution (89%), (Merck) |
| Phytin Acid | Hexa-Phosphoric acid (40 % solution in water); (Sigma-Aldrich, St. Louis, MO) |

### POLYACID A

### Poly(acrylic) acid

An aqueous solution of acrylic acid (Interorgana GmbH & Co. KG, 50668 Koeln, Germany) and ammonium persulfate (Peroxid Chemie GmbH, 82049 Pullach, Germany, 99 %) (500:1 molar ratio) was heated to 80°C over 4 hours. After cooling to room temperature, the solution was purified by membrane filtration and isolated by spray drying to afford a powder that was characterized as poly(acrylic) acid having an average molecular weight (MW) of 50,000 - 60,000 g/mol.

### Filler B

QUARTZ FILLER Surface-Modified with POLYACID A QUARTZ FILLER (200 g), APS (10 g), and DMF (1000 g) were mixed in a round-bottom flask and heated to 120°C with stirring for 17 hours after which DMF (400 ml) was removed on a rotary evaporator under reduced pressure. To the resulting concentrated slurry was added POLYACID A (84 g) and the resulting mixture was heated to 130°C for 13 hours. The remaining DMF was removed with a vacuum rotary evaporator add the residue was treated four times with water and centrifuged. After removal of the aqueous extract, the resulting solid was dried at 90°C under vacuum over night. The solid was ground in a mortar and sieved (Mesh 42 µm) to afford a white powder that was designated Filler B. The yield was 190.1 g.

### Example 1

### RMGI Composition without HEMA

An RMGI composition was prepared by combining Paste A1 (combined ingredients as listed in Table 1) with Paste B1 (combined ingredients as listed in Table 2) in a weight ratio of Paste A1/Paste B1 = 1.0:1.0. The pastes mixed easily and the resulting paste composition (Example 1) had a good consistency. The composition was light cured for 40 seconds using an ELIPAR TRILIGHT curing light (3M ESPE) to afford a hardened material that was evaluated for Flexural Strength according to the test method described herein. Results were as follows:
Flexural Strength: 63 ± 5 MPa (average and SD from 5 replicates)

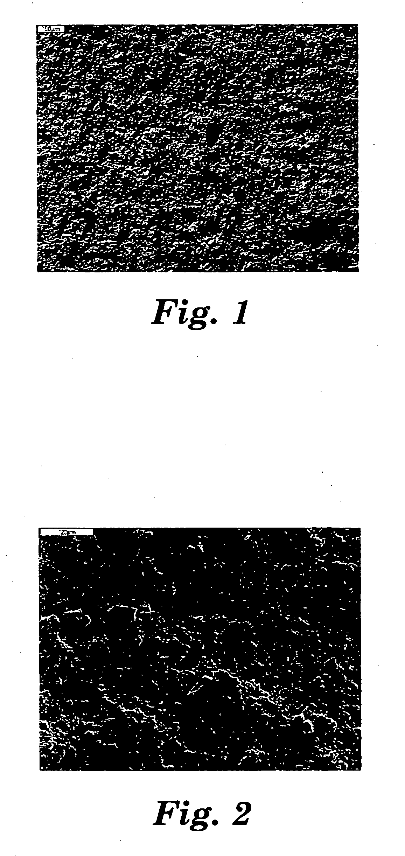

The hardened material (fractured samples) was evaluated by SEM according to the test method described herein and no separation of the GI salt matrix and the resin matrix was observed, as shown in Figs. 1 and 2.

The uncured pastes (A1 and B1) did not show any visual indication of component separation during storage at ambient temperature (23°C, > 95 % relative humidity) over a period of at least 6 months.

| Table 1 | |
|---|---|
| Paste A1 Composition - Weight Percent of Components | |
| Component | Paste A1 |
| TPO-L | 0.30 |
| UDMA | 20.93 |
| Ebecryl 168 | 4.17 |
| Filler A (FAS Glass) | 74.60 |
| Total: | 100 |

| Table 2 | | | |
|---|---|---|---|
| Paste B Compositions - Weight Percent of Components | | | |
| Component | Paste B1 | Paste B2 | Paste B3 |
| POLYACID A (Powder) | 18.85 | 17.7 | 0 |
| POLYACID A in Water (49.3% by weight) | 0 | 0 | 37.7 |
| Water | 18.85 | 18.2 | 0 |
| Filler B ( Polyacid-Modified Surface) | 10.00 | 10.00 | 10.00 |
| Filler C (Silane-Modified Surface) | 50.3 | 50.3 | 50.3 |
| Phosphoric Acid (89% by weight) | 2.00 | 0 | 0 |
| UDMA | 0 | 3.86 | 0 |
| Phytin Acid | 0 | 0 | 2.00 |
| Total: | 100 | 100 | 100 |

### Example 2

### RMGI Composition without HEMA

An RMGI composition was prepared by combining Paste A1 (combined ingredients as listed in Table 1) with Paste B2 (combined ingredients as listed in Table 2) in a weight ratio of Paste A1/Paste B2 = 1.0:1.0. The pastes mixed easily and the resulting paste composition (Example 2) had a good consistency. The composition was light cured for 40 seconds using an ELIPAR TRILIGHT curing light to afford a hardened material that was evaluated for Flexural Strength according to the test method described herein. Results were as follows:
Flexural Strength: 62 ± 8 MPa (average of 5 replications)

The uncured pastes (A1 and B2) did not show any visual indication of component separation during storage at ambient temperature (23°C, > 95 % relative humidity) over a period of at least 6 months.

### Example 3

### RMGI Composition without HEMA

An RMGI composition was prepared by combining Paste A1 (combined ingredients as listed in Table 1) with Paste B3 (combined ingredients as listed in Table 2) in a weight ratio of Paste A1/Paste B3 = 1.0:1.0. The pastes mixed easily and the resulting paste composition (Example 3) had a good consistency. The composition was light cured for 40 seconds using an EUPAR TRILIGHT curing light to afford a hardened material that was evaluated for Flexural Strength and Adhesion to Dentin according to the test methods described herein. Results were as follows:
Flexural Strength: 65 ± 9 MPa (average and SD from 5 replicates)
Adhesion to Dentin (without chemical pretreatment): 0.3 ± 0.4 MPa
Adhesion to Dentin (with Prompt-L-Pop (3M ESPE) pretreatment): 3.7 ± 2.0 MPa

The uncured pastes (A1 and B3) did not show any visual indication of component separation during storage at ambient temperature (23°C, > 95 % relative humidity) over a period of at least 6 months.

### Example 4

### Comparative Studies

An RMGI composition without HEMA (Example 3) was compared to the following commercial products (all of which contained a HEMA component) and that were mixed and hardened according to manufacturer's instructions: PHOTAC-FIL QUICK APLICAP Light-Curing Glass lonomer Restorative Material (3M ESPE), VITREMER Light-Curing and Self-Curing Glass lonomer Restorative Material (3M ESPE), GC FUJI II LC (Hand Mixed; GC Corp.), and GC FUJI II LC (Capsule Mixed; GC Corp.). The results of evaluations of these materials according to the test methods provided herein are shown in Table 3.

| Table 3 | | | | | |
|---|---|---|---|---|---|
| Example 3 Comparison with Commercial Restorative Products | | | | | |
| Physical or Chemical Property | PHOTAC-FIL QUICK APLICAP | VITREMER | GC FUJI II LC (Hand Mixed) | GC FUJI II LC (Capsule Mixed) | Example 3 |
| HEMA Content | Contains HEMA | Contains HEMA | Contains HEMA | Contains HEMA | No HEMA |
| Powder/Liquid Ratio (weight/weight) | 3.1 | 2.5 | 3.2 | 3.2 | - |
| Paste/Paste Ratio (weight/weight) | - | - | - | - | 1.0/1.0 |
| Flexural Strength (MPa ± SD) | 57.0 ± 5.0 | 57.7 ± 9.9 | 62.6 ± 11.1 | 56.1 ± 11.5 | 6.5 ± 9 |
| Adhesion to Dentin (MPa ± SD) (With Pretreatment as Indicated) | 5.1 ± 1.2 (None) | 3.0 ± 0.8 (with VITREMER) Primer) | 3.5 ± 1.8 (with GC Conditioner) | 4.7 ± 3.5 (with GC Conditioner) | 3.7 ± 2.0 (with Prompt-L-Pop) |
| Adhesion to Dentin (MPa ± SD) (Without Pretreatment) | 5.1 ± 1.2 | 0 | 1.7 ± 0.8 | 1.9 ± 1.2 | 0.4 ± 0.4 |

## Claims

1. A hardenable dental composition comprising:
(a) a polymerizable component comprising an ethylenically unsaturated compound; and
(b) a first polyacid that is bonded to the surface of a filler.

2. The hardenable dental composition of claim 1, further comprising;
(c) a second polyacid;
(d) an acid-reactive filler; and
(e) water. ,

3. The composition of claim 2, wherein the first polyacid is selected from the group consisting of homopolymers and copolymers of acrylic acid, maleic acid, itaconic acid, methacrylic acid, and combinations thereof.

4. The composition of claim 2, wherein the second polyacid comprises a polymer having a plurality of acidic repeating units selected from the group consisting of carboxylic acids, sulfuric acids, sulfonic acids, phosphoric acids, phosphonic acids and combinations therof.

5. The composition of claim 1, wherein the polymerizable component comprises an ethylenically unsaturated compound with acid functionality.

6. The composition of claim 2, wherein the second polyacid comprises a polymer having a plurality of acidic repeating groups but is substantially free of polymerizable groups.

7. The composition of claim 2, wherein the first polyacid and second polyacid are the same.

8. The composition of claim 5, wherein the first and/or second polyacid and the ethylenically unsaturated compound with acid functionality are the same.

9. The composition of claim 2, wherein the acid-reactive filler is selected from the group consisting of metal oxides, glasses, glass ceramics, metal salts, and combinations thereof.

10. The composition of claim 9, wherein the acid-reactive filler comprises a fluoroaluminosilicate (FAS) glass.

11. The composition of claim 2, wherein the acid-reactive filler comprises an oxyfluoride material.

12. The composition of claim 2, wherein the composition is a resin-modified glass ionomer that sets after mixing to a homogeneous paste, without showing phase separation, and is substantially free of 2-hydroxyethyl methacrylate ("HEMA").

13. The composition of claim 2, wherein the first polyacid is bonded to the filler via a linking group.

14. The composition of claim 13, wherein the linking group is selected from the group consisting of aminoalkyltrialkoxysilanes.

15. The composition of claim 14, wherein the aminoalkyltrialkoxysilane linking group is attached to the polyacid via an amido moiety and is attached to the filler via a silane atom.

16. The composition of claim 2, further comprising a redox cure system.

17. The composition of claim 2, further comprising a photoinitiator system.

18. The composition of claim 2, further comprising at least one additive selected from the group consisting of other fillers, pyrogenic fillers, fluoride sources, whitening agents, anticaries agents, antiplaque agents, remineralizing agents, enzymes, breath fresheners, anesthetics, clotting agents, acid
neutralizers, chemotherapeutic agents, immune response modifiers, medicaments, indicators, dyes, pigments, wetting agents, tartaric acid, chelating agents, surfactants, buffering agents, viscosity modifiers, thixotropes, polyols, antimicrobial agents, anti-inflammatory agents, antifungal agents, stabilizers, agents for treating xerostomia, desensitizers, and combinations thereof.

19. The composition of claim 2, wherein the composition is selected from the group consisting of dental restoratives, dental adhesives, dental cements, cavity liners, orthodontic adhesives, dental sealants, and dental coatings.

20. The composition of claim 2, wherein the composition is a multi-part composition comprising a first part and a second part, wherein each part can independently be selected from the group consisting of a liquid, paste, gel, and powder.

21. The composition of claim 20, wherein the multi-part composition is selected from the group consisting of a paste-paste composition, a paste-liquid composition, a paste-powder composition, and a powder-liquid composition including multiple combinations thereof.

22. The composition of claim 2 being multi-part hardenable dental composition comprising:
(a) a first part comprising a first polyacid;
(b) a second part comprising an acid-reactive filler;
(c) a second polyacid that is bonded to a filler, the second polyacid present in the first part;
(d) a polymerizable component comprising an ethylenically unsaturated compound present in the first part, the second part, or both parts; and
(e) water present in the first part.

23. The composition of claim 22, wherein the first part comprises a paste.

24. The composition of claim 22, wherein the second part comprises a paste.

25. The composition of claim 22, wherein the first part and the second part each comprises a paste.

26. The hardenable dental composition of claim 1 comprising a filler comprising a particle having a polyacid component bonded to its surface, wherein the particle comprises oxides of silicon, titanium, aluminum, cerium, tin, yttrium, strontium, barium, lanthanum, zinc, ytterbium, bismuth, iron, antimony, or combinations thereof, and a polymerizable component comprising an ethylenically unsaturated compound.

27. The composition of claim 26, wherein the filler comprises a silica particle.

28. The composition of claim 26, wherein the acidic component is a polyacid.

29. The composition of claim 26, wherein the composition is a resin-modified glass ionomer composition.

30. A dental adhesive comprising a composition of any of claims 1 - 29.

31. A dental restorative comprising a composition of any of claims 1 - 29.

## Patentansprüche

1. Härtbare Dentalmasse, umfassend:
(a) eine polymerisierbare Komponente, die eine ethylenisch ungesättigte Verbindung umfaßt; und
(b) eine erste Polysäure, die an die Oberfläche eines Füllstoffs gebunden ist.

2. Härtbare Dentalmasse nach Anspruch 1, ferner umfassend:
(c) eine zweite Polysäure;
(d) einen säurereaktiven Füllstoff und
(e) Wasser.

3. Masse nach Anspruch 2, wobei die erste Polysäure aus der Gruppe bestehend aus Homopolymeren und Copolymeren von Acrylsäure, Maleinsäure, Itaconsäure, Methacrylsäure und Kombinationen davon ausgewählt ist.

4. Masse nach Anspruch 2, wobei die zweite Polysäure ein Polymer mit mehreren sauren Wiederholungseinheiten aus der Gruppe bestehend aus Carbonsäuren, Schwefelsäuren, Sulfonsäuren, Phosphorsäuren, Phosphonsäuren und Kombinationen davon umfaßt.

5. Masse nach Anspruch 1, wobei die polymerisierbare Komponente eine ethylenisch ungesättigte Verbindung mit Säurefunktionalität umfaßt.

6. Masse nach Anspruch 2, wobei die zweite Polysäure ein Polymer mit mehreren sauren Wiederholungseinheiten umfaßt, aber weitgehend frei von polymerisierbaren Gruppen ist.

7. Masse nach Anspruch 2, wobei die erste Polysäure und die zweite Polysäure identisch sind.

8. Masse nach Anspruch 5, wobei die erste und/oder zweite Polysäure und die ethylenisch ungesättigte Verbindung mit Säurefunktionalität identisch sind.

9. Masse nach Anspruch 2, wobei der säurereaktive Füllstoff aus der Gruppe bestehend aus Metalloxiden, Gläsern, Glaskeramiken, Metallsalzen und Kombinationen davon ausgewählt ist.

10. Masse nach Anspruch 9, wobei der säurereaktive Füllstoff ein Fluoroaluminosilicatglas (FAS-Glas) umfaßt.

11. Masse nach Anspruch 2, wobei der säurereaktive Füllstoff ein Oxidfluoridmaterial umfaßt.

12. Masse nach Anspruch 2, wobei es sich bei der Masse um ein harzmodifiziertes Glasionomer handelt, das nach Mischen zu einer homogenen Paste fest wird, ohne Phasentrennung zu zeigen, und weitgehend frei von 2-Hydroxyethylmethacrylat ("HEMA") ist.

13. Masse nach Anspruch 2, wobei die erste Polysäure über eine Brückengruppe an den Füllstoff gebunden ist.

14. Masse nach Anspruch 13, wobei die Brückengruppe aus der Gruppe bestehend aus Aminoalkyltrialkoxysilanen ausgewählt ist.

15. Masse nach Anspruch 14, wobei die Aminoalkyltrialkoxysilan-Brückengruppe über eine Amidogruppierung an die Polysäure gebunden ist und über ein Silanatom an den Füllstoff gebunden ist.

16. Masse nach Anspruch 2, ferner umfassend ein Redox-Härtungssystem.

17. Masse nach Anspruch 2, ferner umfassend ein Photoinitiatorsystem.

18. Masse nach Anspruch 2, ferner umfassend mindestens ein Additiv aus der Gruppe bestehend aus anderen Füllstoffen, pyrogenen Füllstoffen, Fluoridquellen, Bleichmitteln, Antikariesmitteln, Antiplaquemitteln, Remineralisierungsmitteln, Enyzmen, Atemerfrischungsmitteln, Anästhetika, Gerinnungsmitteln, Säureneutralisationsmitteln, Chemotherapeutika, Mitteln zur Modifizierung der Immunantwort, Arzneimitteln, Indikatoren, Farbstoffen, Pigmenten, Netzmitteln, Weinsäure, Chelatbildnern, Tensiden, Puffersubstanzen, Viskositätsmodifikatoren, Thixotropen, Polyolen, antimikrobiellen Mitteln, Antiphlogistika, Antimykotika, Stabilisatoren, Mitteln zur Behandlung von Xerostomie, Desensibilisatoren und Kombinationen davon.

19. Masse nach Anspruch 2, wobei die Masse aus der Gruppe bestehend aus Dentalrestaurationsmitteln, Dentalklebstoffen, Dentalzementen, Kavitätsauskleidungen, kieferorthopädischen Klebstoffen, Dentalversiegelungsmitteln und Dentalbeschichtungen ausgewählt ist.

20. Masse nach Anspruch 2, wobei es sich bei der Masse um eine mehrteilige Masse handelt, die einen ersten und einen zweiten Teil umfaßt, wobei jeder Teil unabhängig voneinander aus der Gruppe bestehend aus einer Flüssigkeit, einer Paste, einem Gel und einem Pulver ausgewählt sein kann.

21. Masse nach Anspruch 20, wobei die mehrkomponentige Masse aus der Gruppe bestehend aus einer Paste-Paste-Masse, einer Paste-Flüssigkeit-Masse, einer Paste-Pulver-Masse und einer Pulver-Flüssigkeit-Masse einschließlich multiplen Kombinationen davon ausgewählt ist.

22. Masse nach Anspruch 2, bei der es sich um eine mehrteilige härtbare Dentalmasse, umfassend:
(a) einen ersten Teil, der eine erste Polysäure umfaßt;
(b) einen zweiten Teil, der einen säurereaktiven Füllstoff umfaßt;
(c) eine zweite Polysäure, die an einen Füllstoff gebunden ist, wobei die zweite Polysäure im ersten Teil vorliegt;
(d) eine polymerisierbare Komponente, die eine ethylenisch ungesättigte Verbindung umfaßt und im ersten Teil, im zweiten Teil oder in beiden Teilen vorliegt; und
(e) Wasser, das im ersten Teil vorliegt;
handelt.

23. Masse nach Anspruch 22, wobei der erste Teil eine Paste umfaßt.

24. Masse nach Anspruch 22, wobei der zweite Teil eine Paste umfaßt.

25. Masse nach Anspruch 22, wobei der erste Teil und der zweite Teil jeweils eine Paste umfassen.

26. Härtbare Dentalmasse nach Anspruch 1, umfassend einen Füllstoff, umfassend ein Teilchen mit einer an dessen Oberfläche gebundenen Polysäurekomponente, wobei das Teilchen Oxide von Silicium, Titan, Aluminium, Cer, Zinn, Yttrium, Strontium, Barium, Lanthan, Zink, Ytterbium, Bismut, Eisen, Antimon oder Kombinationen davon umfaßt, und eine polymerisierbare Komponente, die eine ethylenisch ungesättigte Verbindung umfaßt.

27. Masse nach Anspruch 26, wobei der Füllstoff ein Siliciumdioxidteilchen umfaßt.

28. Masse nach Anspruch 26, wobei es sich bei der sauren Komponente um eine Polysäure handelt.

29. Masse nach Anspruch 26, wobei es sich bei der Masse um eine harzmodifizierte Glasionomermasse handelt.

30. Dentalklebstoff, umfassend eine Masse nach einem der Ansprüche 1-29.

31. Dentalrestaurationsmittel, umfassend eine Masse nach einem der Ansprüche 1-29.

## Revendications

1. Composition dentaire durcissable comprenant :
(a) un composant polymérisable comprenant un composé à insaturation éthylénique ; et
(b) un premier polyacide qui est lié à la surface d'une charge.

2. Composition dentaire durcissable selon la revendication 1, comprenant en outre :
(c) un second polyacide ;
(d) une charge réactive à l' acide ; et
(e) de l'eau.

3. Composition selon la revendication 2, dans laquelle le premier polyacide est choisi parmi le groupe constitué d'homopolymères et de copolymères de l'acide acrylique, de l'acide maléique, de l'acide itaconique, de l'acide méthacrylique et de combinaisons de ceux-ci.

4. Composition selon la revendication 2, dans laquelle le second polyacide comprend un polymère qui possède une pluralité de motifs répétés acides choisis parmi le groupe constitué d'acides carboxyliques, d'acides sulfuriques, d'acides sulfoniques, d'acides phosphoriques, d'acides phosphoniques et de combinaisons de ceux-ci.

5. Composition selon la revendication 1, dans laquelle le composant polymérisable comprend un composé à insaturation éthylénique avec une fonctionnalité acide.

6. Composition selon la revendication 2, dans laquelle le second polyacide comprend un polymère qui comprend une pluralité de motifs répétés acides, mais qui est essentiellement exempt de groupes polymérisables.

7. Composition selon la revendication 2, dans laquelle le premier polyacide et le second polyacide sont identiques.

8. Composition selon la revendication 5, dans laquelle le premier et/ou le second polyacide et le composé à insaturation éthylénique avec une fonctionnalité acide sont identiques.

9. Composition selon la revendication 2, dans laquelle la charge réactive à l'acide est choisie parmi le groupe constitué d'oxydes métalliques, de verres, de céramiques de verre, de sels métalliques et de combinaisons de ceux-ci.

10. Composition selon la revendication 9, dans laquelle la charge réactive à l'acide comprend un verre de fluoroaluminosilicate (FAS).

11. Composition selon la revendication 2, dans laquelle la charge réactive à l'acide comprend un matériau d'oxyfluorure.

12. Composition selon la revendication 2, dans laquelle la composition est un ionomère de verre modifié par une résine qui durcit après un mélange avec une pâte homogène, sans présenter de séparation de phase, et est essentiellement exempte de méthacrylate de 2-hydroxyéthyle (« HEMA »).

13. Composition selon la revendication 2, dans laquelle le premier polyacide est lié à la charge par un groupe de liaison.

14. Composition selon la revendication 13, dans laquelle le groupe de liaison est choisi parmi le groupe constitué d'aminoalkyltrialcoxysilanes.

15. Composition selon la revendication 14, dans laquelle le groupe de liaison aminoalkyltrialcoxysilane est attaché au polyacide par un groupement amido et est attaché à la charge par un silane.

16. Composition selon la revendication 2, qui comprend en outre un système redox de durcissement.

17. Composition selon la revendication 2, qui comprend en outre un système photo-initiateur.

18. Composition selon la revendication 2, qui comprend en outre au moins un additif choisi parmi le groupe constitué d'autres charges, de charges pyrogènes, de sources de fluorure, d'agents de blanchiment, d'agents anticaries, d'agents antiplaques, d'agents de reminéralisation, d'enzymes, de rafraîchisseurs d'haleine, d'anesthésiques, d'agents de coagulation, de neutralisants d'acide, d'agents chimiothérapiques, de modificateurs de la réponse immune, de médicaments, d'indicateurs, de colorants, de pigments, d'agents mouillants, d'acide tartrique, d'agents chélatants, d'agents tensio-actifs, d'agents tampons, de modificateurs de viscosité, de thixotropes, de polyols, d'agents antimicrobiens, d'agents anti-inflammatoires, d'agents antifongiques, de stabilisants, d'agents pour traiter la xérostomie, de désensibilisants et de combinaisons de ceux-ci.

19. Composition selon la revendication 2, dans laquelle la composition est choisie parmi le groupe constitué de produits pour restauration dentaire, d'adhésifs dentaires, de ciments dentaires, de fonds de cavité, d'adhésifs orthodontiques, de résines de scellement et de revêtements dentaires.

20. Composition selon la revendication 2, dans laquelle la composition est une composition en plusieurs parties qui comprend une première partie et une seconde partie, dans laquelle chaque partie peut indépendamment être choisie parmi le groupe constitué d'un liquide, d'une pâte, d'un gel et d'une poudre.

21. Composition selon la revendication 20, dans laquelle la composition en plusieurs parties est choisie parmi le groupe constitué d'une composition pâte-pâte, d'une composition pâte-liquide, d'une composition pâte-poudre et d'une composition poudre-liquide, y compris de multiples combinaisons de celles-ci.

22. Composition selon la revendication 2 qui est une composition dentaire durcissable en plusieurs parties qui comprend :
(a) une première partie comprenant un premier polyacide ;
(b) une seconde partie comprenant une charge réactive à l'acide ;
(c) un second polyacide qui est lié à une charge, le second polyacide étant présent dans la première partie ;
(d) un composant polymérisable comprenant un composé à insaturation éthylénique présent dans la première partie, la seconde partie ou les deux parties ; et
(e) de l'eau présente dans la première partie.

23. Composition selon la revendication 22, dans laquelle la première partie comprend une pâte.

24. Composition selon la revendication 22, dans laquelle la seconde partie comprend une pâte.

25. Composition selon la revendication 22, dans laquelle la première partie et la seconde partie comprennent chacune une pâte.

26. Composition dentaire durcissable selon la revendication 1 comprenant une charge qui comprend une particule qui possède un composant polyacide lié à sa surface, dans laquelle la particule comprend des oxydes de silicium, de titane, d'aluminium, de cérium, d'étain, d'yttrium, de strontium, de baryum, de lanthane, de zinc, d'ytterbium, de bismuth, de fer, d'antimoine ou de combinaisons de ceux-ci, et un composant polymérisable comprenant un composé à insaturation éthylénique.

27. Composition selon la revendication 26, dans laquelle la charge comprend une particule de silice.

28. Composition selon la revendication 26, dans laquelle le composant acide est un polyacide.

29. Composition selon la revendication 26, dans laquelle la composition est une composition ionomère de verre modifié par une résine.

30. Adhésif dentaire comprenant une composition selon l'une quelconque des revendications 1 à 29.

31. Matériau de restauration dentaire comprenant une composition selon l'une quelconque des revendications 1 à 29.
